# EUROPEAN PATENT SPECIFICATION

(11) **EP 3 664 857 B1**
(45) Date of publication and mention of the grant of the patent: **21.12.2022**
(21) Application number: 18773924.8
(22) Date of filing: 17.07.2018
(51) Int. Cl.: A61L 27/20, A61L 27/24, A61L 27/26, A61L 31/04

(54) **A PROCESS FOR PRODUCING A TWO-STAGE CROSSLINKED BIOPOLYMER MEMBRANE AND A BIOPOLYMER MEMBRANE MADE IN THIS PROCESS**
VERFAHREN ZUR HERSTELLUNG EINER ZWEISTUFIGEN VERNETZTEN BIOPOLYMERMEMBRAN UND IN DIESEM VERFAHREN HERGESTELLTE BIOPOLYMERMEMBRAN
PROCÉDÉ POUR LA PRODUCTION D'UNE MEMBRANE EN BIOPOLYMÈRES RÉTICULÉE EN DEUX ÉTAPES ET MEMBRANE EN BIOPOLYMÈRES FABRIQUÉE DANS CE PROCÉDÉ

(30) Priority: 20.07.2017 CZ 20170421
(43) Date of publication of application: 17.06.2020
(73) Proprietor: Tomas Bata University In Zlín, 76001 Zlín (CZ)
(72) Inventor: RUSHITA JASWANT, Shah, Gujarat (IN); SAHA, Petr, 76001 Zlin Mladcova (CZ); STODULKA, Pavel, 76001 Zlin Priluky (CZ)
(74) Representative: Kreizlova, Dana
(86) International application number: PCT/CZ2018/050039
(87) International publication number: WO 2019/015704

(56) References cited:
- US-A1- 2014 142 200
- US-A1- 2017 142 967
- MARIA GROLIK ET AL: "Hydrogel membranes based on genipin-cross-linked chitosan blends for corneal epithelium tissue engineering", JOURNAL OF MATERIALS SCIENCE: MATERIALS IN MEDICINE, KLUWER ACADEMIC PUBLISHERS, BO, vol. 23, no. 8, 9 May 2012 (2012-05-09), pages 1991-2000, XP035087810, ISSN: 1573-4838, DOI: 10.1007/S10856-012-4666-7
- YAO C H ET AL: "CROSS-LINKING TREATMENT OF COLLAGENOUS IMPLANTS BY CHEMICAL METHODS", BIOMEDICAL ENGINEERING - APPLICATIONS, BASIS & COMMUNICATIONS, XX, XX, vol. 10, no. 3, 1 June 1998 (1998-06-01), pages 156-164, XP000913442, ISSN: 1016-2356
- PUNITHA VELMURUGAN ET AL: "Investigation on interaction of tannic acid with type I collagen and its effect on thermal, enzymatic, and conformational stability for tissue engineering applications : Investigation on Interaction of Tannic Acid with Collagen", BIOPOLYMERS, vol. 101, no. 5, 21 February 2014 (2014-02-21), pages 471-483, XP055526547, US ISSN: 0006-3525, DOI: 10.1002/bip.22405
- RUBENTHEREN V ET AL: "Effects of heat treatment on chitosan nanocomposite film reinforced with nanocrystalline cellulose and tannic acid", CARBOHYDRATE POLYMERS, APPLIED SCIENCE PUBLISHERS, LTD. BARKING, GB, vol. 140, 29 December 2015 (2015-12-29), pages 202-208, XP029417161, ISSN: 0144-8617, DOI: 10.1016/J.CARBPOL.2015.12.068
- DATABASE WPI Week 201611 Thomson Scientific, London, GB; AN 2015-772154 XP002786780, & CN 105 034 369 A (QINGDAO UNIQUE TECHNOLOGY CO LTD) 11 November 2015 (2015-11-11)
- RAFAT M ET AL: "PEG-stabilized carbodiimide crosslinked collagen-chitosan hydrogels for corneal tissue engineering", BIOMATERIALS, ELSEVIER SCIENCE PUBLISHERS BV., BARKING, GB, vol. 29, no. 29, 1 October 2008 (2008-10-01), pages 3960-3972, XP023611041, ISSN: 0142-9612, DOI: 10.1016/J.BIOMATERIALS.2008.06.017 [retrieved on 2008-07-21]

## Description

### Field of technology

The present invention relates to a process of fabrication of a two-step/double cross-linked biopolymer membrane and a biopolymer membrane made in this manner for the repair of ocular traumas, in particular for repair of corneal trauma.

### Background of the invention

Ocular diseases and wounds affect millions of people worldwide. In most cases they are diseases or injuries affecting the cornea. In recent years, a lot of attention has been given to engineering biopolymers of three-dimensional structure for creating scaffolds for tissue engineering. Bio-interactive scaffolds are gaining importance in regenerative medicines, as they mimic the extracellular matrix (ECM) of the body. A wide range of biomaterials prepared comprises of hybrids of natural and synthetic polymers and thereby mimicking the extracellular matrix components. These biomimetic materials are fabricated in form of hydrogels, membranes, meshes and other structures.

Researchers have already reported several biomaterials prepared from biopolymers, natural cross-linkers for ocular therapeutics and tissue repair. Chitosan, the de-acetylated form of chitin, is currently frequently utilized in various medical and pharmaceutical applications because of its good biocompatibility, inertness, film forming ability, sterilizable non-toxic and biodegradable in nature.

Collagen matrix, a successful resorbable biomaterial used in tissue engineering, exhibits good hemostatic, low antigenicity (ability to elicit an immune response) and inflammatory properties.

Plant polyphenols like tannic acid are categorized as an astringent, antioxidant, antimicrobial, antiviral, and anti-inflammatory agents also serving as cross-linking agents for collagen-based membranes.

Genipin obtained from natural source Gardenia jasminoides Ellis, serves as an effective cross-linking agent in polymers with amino group.

Currently, researchers (J. Jeremy Chae et.al. 2015) have reconstructed corneal epithelium using collagen membrane which is essential for the healing process. Type I collagen was used, imparting beneficial epithelial cell migration and proper differentiation.

The entire study was conducted on rabbits.

Also, the in vivo biocompatibility study of genipin cross-linked chitosan membrane was performed on the anterior chamber of the eyes of rabbits (Jui-Yang Lai , 2012).

The patent US 6624138 relates to the material prepared by cross-linking biological substances, such as collagen, chitosan, with genipin, a naturally occurring cross-linking agent claiming for wound dressing or adhesive.

The subject of US 8642088 patent is mixing of matrix of chitosan and tannin for drug delivery, antibacterial and/or antifungal, tissue engineering, wound healing applications.

The patent application US 2014142200 describes the double cross-linked stage of collagen materials for the application in ophthalmology as an ophthalmic device (especially in keratoprosthesis implantation) in form of molds. Here, chemical cross-linkers like EDC (lethyl-3 '-(3-dimethylaminopropyl) carbodiimide as primary cross-linker, along with NHS (N-hydroxy succinimide) as secondary cross-linker are utilized.

M. Grolik et al. disclose a method of preparing hydrogel membranes as scaffolds for corneal tissue engineering, comprising mixing solutions of collagen and chitosan with genipin and casting on a plastic dish (JOURNAL OF MATERIAL SCIENCE: MATERIALS IN MEDICINE, 23(8), 09-05-2012, 1991-2000, XP035087810).

Until now, there exists no report wherein two types of natural cross-linkers together, cross-linked the polymeric membrane consecutively thus forming thin polymeric membrane. The membrane obtained can serve as a temporary implant to the injured cornea or due to irregularity in the anterior layer of cornea caused by some corneal disease.

### Grounds of the technical solution

The method of fabrication of a double cross-linked biopolymer membrane for correcting eye trauma according to the invention contributes to removal of the above listed deficiencies.

The fabrication of a double/two-step cross-linked biopolymer membrane for corneal trauma repair lies in preparation of a mixed solution of biopolymers consisting of collagen and chitosan, dropwise adding a solution of the primary crosslinking agent from the group of tannins and pouring the solution onto a flat pad and then drying to form a thin primary cross-linked chitosan-collagen membrane with a thickness after drying of 5 to 10 µm. This is then cross-linked in the second stage with a solution of genipin as a secondary crosslinking agent.

A mixed solution of collagen-chitosan biopolymers is a solution in acetic acid with total concentration of the mixture of biopolymers of 0,5 to 2% by weight and a weight ratio of collagen to chitosan of 20:80 up to 30:70.

As a crosslinking agent from the groups of tannins the tannic acid will be used in demineralized water of 0, 5 to 3 % concentration.

As a secondary crosslinking agent a 0,25 to 0,5% genipin solution in dimethyl-sulphoxid (DMSO) with phosphate buffer will be used

The use of a mixture of collagen and chitosan biopolymers and a combination of two natural crosslinking agents represents a new way of producing a biomaterial with a presumptive application in ophthalmology. In this process of producing a double cross-linked membrane, hydrogen bonding is primarily formed by the action of tannic acid, followed by a covalent crosslinking between the amino groups of the biopolymer and the secondary cross-linker by genipin. The whole process is carried out at room temperature (25-28 ⁰ C) ranging from 48 to 72 hours. This is a newly formulated process to obtain a non-transparent, darkcolored membrane foil that is applied to the marginal area of the eye during eye surgery.

### Overview of drawings/figures

Closer explanations of the spirit of the invention are given by the accompanying drawings in which the following is represented
Figure I - SEM microscopic images (a) - collagen-chitosan membranes, (b) - collagen-chitosan membranes cross-linked with tannic acid, (c) - collagen-chitosan membranes cross-linked with tannic acid and genipin;

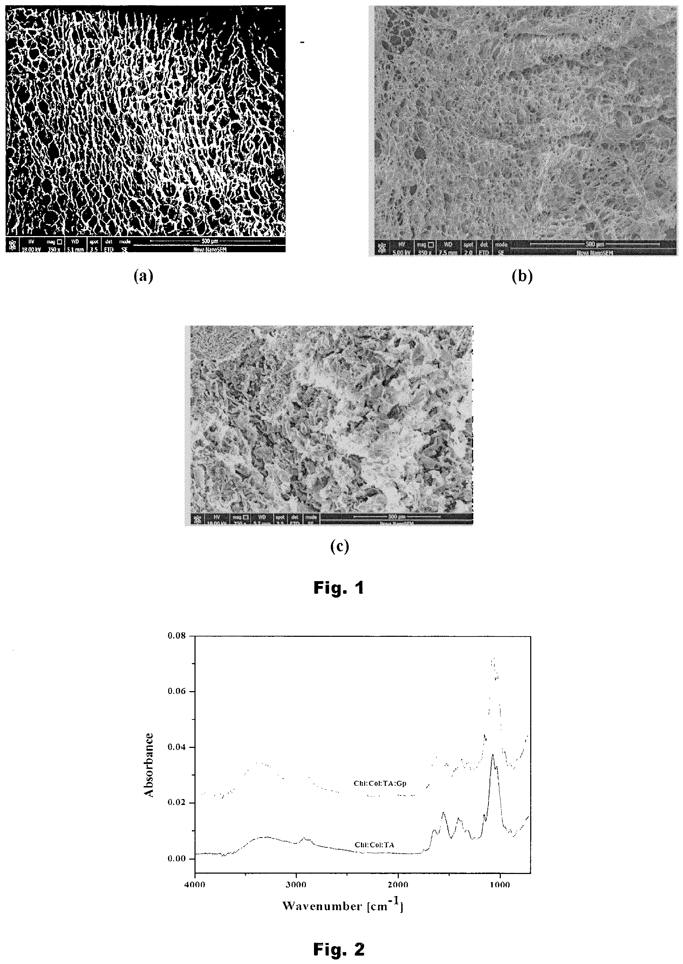

Figure 2 - F TIR collagen-chitosan membrane cross-linked with tannic acid (Chi: Col: TA), collagen-chitosan membranes two-stage cross-linked with tannic acid and genipin (Chi: Col: TA: Gp).

### Examples of implementation of the invention

A mixed solution of the biopolymers chitosan and collagen is prepared in acetic acid (1%-age) of overall concentration of the mixture 0.5 to 2% weight and at a weight ratio of chitosan and collagen 25:75. Under constant magnetic stirring dropwise adding a solution of a primary cross-linker tannic acid in demineralized water at concentration of 0.5-3% Thereafter, the solution is casted onto the polystyrene plates. After drying the suitable layer of thin film (less transparent) is formed with thickness ranging from 5-10 µm along with the transmittance (wavelength at visual range: 400-700nm) 42-48% from the least to most transparent. The film remains porous throughout.

The membrane prepared in stage 1 will then be cross-linked in stage 2 with genipin solution in dimethyl sulphoxide (DMSO) of 0.25-0.5% concentration with phosphate buffer. Following successful cross-linking, genipin-containing networks exhibit a high degree of auto-fluorescence imparting dark greenish -yellow color to the membrane. The thickness of the final developed membrane ranges from 8-10 µm along with the transmittance (around 650nm) < 15% from the least. Also, it persists porosity in its three-dimensional structure.

## Claims

1. A process for producing a two-stage cross-linked biopolymer membrane for corneal trauma repair, being **characterized by** that the process lies in preparation of a mixed solution of biopolymers consisting of collagen and chitosan, dropwise adding a solution of the primary crosslinking agent from the group of tannins and pouring the solution onto a flat pad and then drying to form a thin primary cross-linked chitosan-collagen membrane with a thickness after drying of 5 to 10 µm and this is then cross-linked in the second stage with a solution of genipin as a secondary crosslinking agent.

2. The process according to claim 1, being **characterized by** that mixed solution of collagen-chitosan biopolymers is a solution in acetic acid with total concentration of the mixture of biopolymers of 0, 5 to 2% by weight and a weight ratio of collagen to chitosan of 20:80 up to 30:70.

3. The process according to claim 1, being **characterized by** that tannic acid (tannin) is used as a tannin crosslinking agent..

4. The process according to claim 3, being **characterized by** that as a crosslinking agent from the groups of tannins the tannic acid will be used as a solution in demineralized water of 0.5 to 3% concentration.

5. The process according to claim 1, being **characterized by** that as a secondary crosslinking agent a 0.25 to 0.5% genipin solution in dimethyl-sulphoxid (DMSO) with phosphate buffer will be used.

6. A biopolymer membrane made by the process of claim 1.

## Patentansprüche

1. Verfahren zur Herstellung einer zweistufigen vernetzten Biopolymer-Membrane zur Trauma-reparatur von Hornhaut, **dadurch gekennzeichnet, daß** das Verfahren auf der Zubereitung einer Mischlösung von aus Kollagen und Chitosan bildenden Biopolymeren beruht, in die eine Lösung des primären Vernetzungsmittels aus der Tannin-Gruppe tropfenweise zugegeben wird, und der Lösung auf eine flache Unterlage eingegossen wird und nachfolgende Austrocknung bei der Bildung einer dünnen primärvernetzten Chitosan-Kollagen-Membrane verwirklicht ist, bis zur Dicke von 5 bis 10 µm, die in der zweiten Phase mit Genipin-Lösung vernetzt ist, wobei die Genipin-Lösung als ein sekundäres Vernetzungsmittel bewirkt.

2. Das Verfahren nach dem Anspruch 1, **dadurch gekennzeichnet, daß** die Mischlösung von Kollagen-Chitosan-Biopolymeren eine Lösung der Essigsäure ist, wobei die Gesamtkonzentration der Biopolymermischung 0,5 bis 5 Gewichtsprozent und Gewichtsverhältnis des Kollagens zum Chitosan 20:80 bis 30:70 betragen.

3. Das Verfahren nach dem Anspruch 1, **dadurch gekennzeichnet, daß** als Tannin-Vernetzungsmittel die Tannin-Säure (Tannin) verwendet wird.

4. Das Verfahren nach dem Anspruch 3, **dadurch gekennzeichnet, daß** als ein Vernetzungsmittel von Tannin-Gruppen der Tannin-Säure eine Lösung des demineralisierten Wassers mit der Konzentration von 0,5 bis 3% angewendet wird.

5. Das Verfahren nach dem Anspruch 1, **dadurch gekennzeichnet, daß** als sekundäres Vernetzungsmittel eine Genipin-Lösung in Dimethyl-Sulphoxid (DMSO) mit Phosphatpuffer mit der Konzentration von 0,25 bis 0,5% angewendet wird.

6. Biopolymer-Membrane hergestellte durch das Verfahren nach dem Anspruch 1.

## Revendications

1. Procédé pour la production d'une membrane en biopolymères réticulée en deux étapes pour la réparation de traumatismes cornéens, **caractérisant par** un procédé qui consiste à préparer une solution mixte de biopolymères constituée de collagène et de chitosane, en ajoutant, au goutte à goutte, une solution de l'agent de réticulation primaire du groupe des tanins, en versant la solution sur un tampon plat et en laissant sécher afin qu'une fine membrane de collagène/chitosane réticulée primaire d'une épaisseur après séchage de 5 à 10 µm se forme et que celle-ci soit ensuite réticulée lors de la deuxième étape à l'aide d'une solution de génipine comme agent de réticulation secondaire.

2. Ce procédé, selon la revendication n° 1, **caractérisé en ce qu'**une solution mixte de biopolymères collagène/chitosane est une solution dans l'acide acétique et que la concentration totale du mélange de biopolymères est de 0,5 à 2 % en poids. Le rapport pondéral du collagène au chitosane est de 20/80 à 30/70 maximum.

3. Ce procédé, selon la revendication n° 1, **caractérisé en ce que** l'acide tannique (tanin) est utilisé comme agent de réticulation du tanin.

4. Ce procédé, selon la revendication n° 3, **caractérisé en ce que** l'acide tannique (en tant qu'agent de réticulation des groupes de tannins) est utilisé comme solution dans l'eau déminéralisée d'une concentration de 0,5 à 3 %.

5. Ce procédé, selon la revendication n° 1, **caractérisé en ce qu'**une solution de 0,25 à 0,5 % de génipine dans du diméthylsulfoxyde (DMSO) avec un tampon phosphate est utilisée comme agent de réticulation secondaire.

6. Membrane en biopolymères fabriquée dans ce procédé de la revendication n° 1.
